# EUROPEAN PATENT SPECIFICATION

(11) **EP 1 813 232 B1**
(45) Date of publication and mention of the grant of the patent: **26.08.2009**
(21) Application number: 07250339.4
(22) Date of filing: 27.01.2007
(51) Int. Cl.: A61F 2/84

(54) **Deployment catheter for medical implant devices**
Einführkatheter für medizinische Implantatvorrichtungen
Déploiement de cathéter pour des dispositifs d'implant médical

(30) Priority: 31.01.2006 US 343626
(43) Date of publication of application: 01.08.2007
(73) Proprietor: Cordis Corporation, Miami Lakes, FL 33014 (US)
(72) Inventor: Aruirre, Gus, Pembroke Pines, FL 33029 (US); Cioanta, Iulian, Weston, FL 33327 (US); Couri, Duane, North Miami Beach, FL 33179 (US); Fitzgerald, Eamonn P., Hollywood, FL 33021 (US); Harries, Heather Ann Bartholf, Pembroke Pines, FL 33029 (US)
(74) Representative: Belcher, Simon James

(56) References cited:
- WO-A-00/56248
- WO-A1-99/43379
- US-A1- 2002 010 419
- US-A1- 2002 072 789
- US-B1- 6 183 481

## Description

The present invention relates to the field of medical devices, and more particularly to a method and apparatus for the deployment of medical implant devices, including prosthetic stent devices.

Vascular disease is a leading cause of premature mortality in developed nations, often presenting as a vascular aneurysm. A vascular aneurysm is a localized dilation of a vessel wall, due to thinning or weakness of the wall structure, or separation between layers of the vessel wall. If untreated, the aneurysm may burst and hemorrhage uncontrollably. Aneurysms are particularly dangerous and prevalent in the aorta, because the aorta supplies blood to all other areas of the body, and because the aorta is subject to particularly high pressures and stresses accordingly. Rupture of an aortic aneurysm is the 15^{th} leading cause of death the United States, afflicting 5% of older men.

Aortic aneurysms are described by their position. They are either thoracic, generally between the aortic arch and the junction of the left and right renal arteries, or abdominal, between the junction of the renal arteries and the branch of the iliac arteries. It is known to treat aortic aneurysms surgically where blood pressure control medication is unsuccessful at arresting growth of the aneurysm. Surgery often involves the insertion of a vascular stent graft to exclude the aneurysm and carry blood past the dilated portion of the vessel, relieving the pressure on the aneurysm. Designing a viable stent graft for the treatment of abdominal aortic aneurysm (AAA) is particularly challenging, in part because the graft must branch to follow the shape of the abdominal aorta to carry blood into the separate iliac arteries without obstruction, while preventing continued pressurization of the aneurysm.

Moreover, it would be advantageous to design a stent graft that is collapsible to facilitate percutaneous insertion by minimally invasive surgical techniques. Additionally, percutaneous insertion requires the design and development of a delivery system that can effectively position and deploy the vascular stent.

Towards this end, modular stent grafts have been developed. In certain embodiments of a modular stent graft for treating AAA, a bifurcate first portion is located in the abdominal aorta, while additional portions extend beyond the first portion, for example into the iliac vessels. However, deployment in such vessels has proven challenging. For example, where a separate percutaneous deployment device is used for each stent graft portion, the insertion of the second deployment device after the deployment of the bifurcate first portion can engage the first deployed portion, migrating the first portion in a cranial or cephalid direction. Any migration is considered detrimental. However, in extreme cases, the cranial migration may obstruct blood flow to the renal arteries, a particularly dangerous occurrence.

WO-A-99/43379 discusses a delivery system for a multi-stage stent graft. the system has first and second sheaths for enclosing different stages of the multi-stage stent graft. A first sheath has a narrower portion at its proximal end which is enclosed by the second sheath.

US-A-2002/0072789 discusses a stent delivery system comprising a lubricant application port in a stent retaining sleeve. This allows a lubricant to partially coat a stent positioned under the sleeve.

US-6183481 relates to a stent delivery system for self-expanding stents. Opposing edges of a sheath which surrounds a stent are releasably secured by a release element. When the release element is pulled proximally the edges of the sheath are released, deploying the stent.

WO-A-00/56248 discusses a lubricated sleeve material for stent delivery. The sleeve includes lubricant prior to assembly with a catheter to reduce friction.

US-A-2001/0010419 relates to a balloon catheter provided with a series of sleeve members along the length of the catheter body for receiving a guidewire.

Therefore, in order to overcome these and other deficiencies in the prior art, provided according to the present invention is an apparatus for the delivery and deployment of medical implant devices as defined in appended claim 1. The delivery catheter has a generally cylindrical central core with a distal tip at its end. First and second generally cylindrical pods cover one or more prosthetic implants located around the central core, and are connected with one another to move as a unit and to present a continuous outer surface. The first pod is proximal of the second pod. The second pod is connected with the distal tip to move as a unit and to present a continuous outer surface. First and second independent release links, each extending from a proximal region of the delivery catheter to an interface with the first and second pods, respectively, and operative to displace the first and second pod, respectively.

The first or second independent release links can be secured to the respective first or second pod at a proximal end, or can pass within a respective first or second pod. Applying tension to the release links releases the associated pod, either by displacing it from over the implant or rupturing the exterior of the pod. A user-operable handle at a proximal end of the delivery catheter can activate either or both independent release links.

The present invention can be used in a method for the deployment of a prosthetic implant. One or more implants are crimped to a central core of a delivery catheter, a first implant is proximal of the second. The implants are covered with independent first and second pods. The delivery catheter is inserted into a patient to locate the first implant at a first desired deployment position, and the first pod is displaced from over the first implant, permitting the first implant to expand to a deployed diameter. The delivery catheter is proximally displaced to locate the second implant at a second desired deployment location, and the second pod is displaced from over the second implant, permitting the second stent to expand to a deployed diameter. Independent release links between a proximal region of the deployment catheter and the first or second pods displace the pods by axially sliding the pod in a proximal direction or alternately by rupturing the pod.

Embodiments of the invention will now be described by way of example with reference to the accompanying drawings, wherein like reference numerals refer to like structures across the several views, and in which:
Fig. 1 illustrates a perspective view of a delivery catheter according to a first embodiment of the present invention in an initial configuration;
Fig. 2 illustrates the delivery catheter after deploying a first stent;
Fig. 3 illustrates the delivery catheter following repositioning to deploy a second stent;
Fig. 4 illustrates the delivery catheter after deploying a second stent;
Fig. 5 illustrates the delivery catheter having been withdrawn from the area of deploying the first and second stents;
Fig. 6 illustrates an enlarged view of the delivery catheter, showing primarily a proximal end of the first pod;
Fig. 7 illustrates an enlarged view of the delivery catheter, showing primarily a first pod;
Fig. 8 illustrates an enlarged view of the delivery catheter, showing primarily the interface between a first and second pod; and
Fig. 9 illustrates an enlarged view of the delivery catheter, showing primarily a second pod and distal tip;

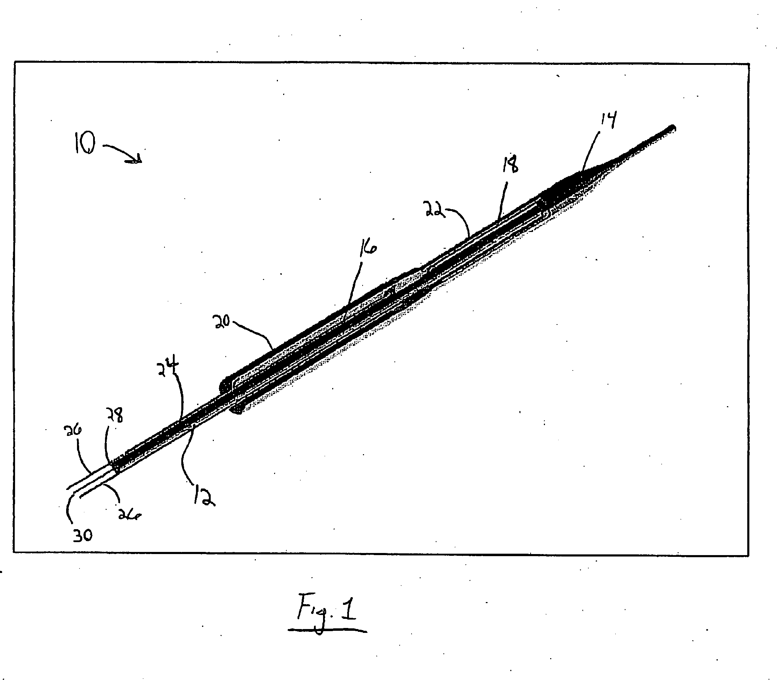

Referring now to Fig. 1, illustrated in perspective view is a delivery catheter, generally 10, for the percutaneous delivery of plural segmented vascular stents, according to a first exemplary embodiment of the present invention. Delivery catheter 10 has a generally cylindrical central core 12 running along its axial length to a distal tip 14. When configured for the delivery of plural segmented vascular stents, a first stent 16 and second stent 18 are both placed over the central core 12 and each is radially compressed or crimped to a delivery diameter smaller than their deployed diameter. First stent 16 and second stent 18 are each surrounded by a sheath or pod 20, 22, that separates the stents 16, 18 from blood in the delivery vessel prior to deployment. Pods 20, 22 are join with one another to present a smooth outer diameter or transition. More preferably, pods 20, 22 are linked to one another to that the delivery catheter 10 may be rotated as a unit.

In the preferred embodiment, stents 16, 18 are designed to and will be deployed to nest at least partially within one another. Specifically, second stent 18 may be designed to nest at least partially within first stent 16. More generally, however, the first stent 16 is preferably deployed in the cranial or cephalid direction from the second stent 18. However, it is undesirable to advance the delivery catheter 10 in the cranial or cephalid direction after the first stent 16 is deployed. Therefore, the second stent 18 is preferably crimped onto the central core 12 of delivery catheter 10 distally from the first stent 16. Accordingly, after deployment of the first stent 16, the delivery catheter 10 is moved in the caudal direction, or proximally, to position the second stent for deployment.

Each pod 20, 22 has an independent release link, 24, 26, respectively. The release link 24, 26 are a hollow, solid or braided filament of metal, or another material, including but not limited to plastic. The release links 24, 26 interface with their respective pods 20, 22 at a distal region of the delivery catheter 10, and extend to a proximal end of the delivery catheter 10 where they may be manipulated by the surgeon.

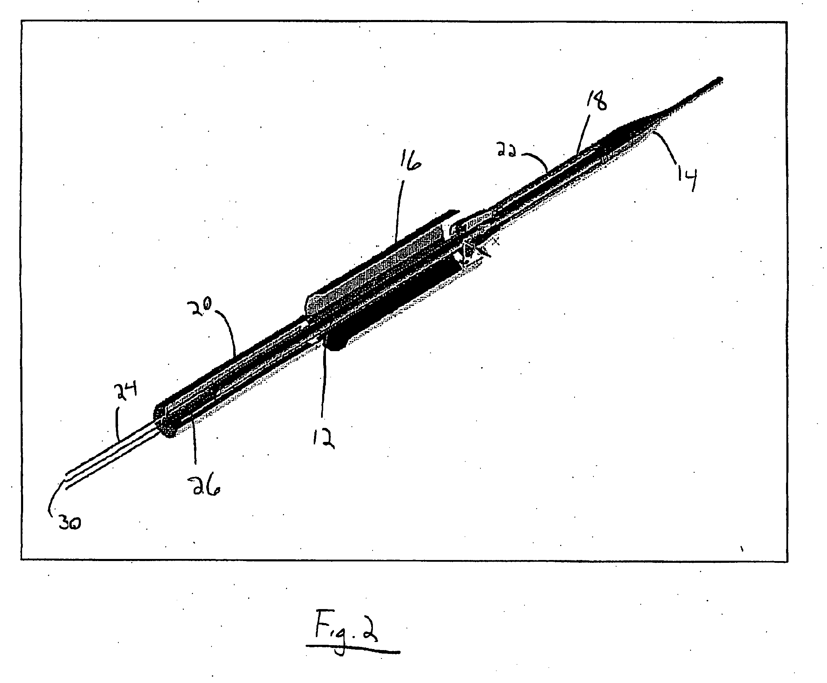

Referring now to Fig. 2, in the exemplary embodiment, the release links 24, 26 are bonded to their respective pods 20, 22 at proximal ends thereof. Once the delivery catheter has positioned the first stent 16 at its deployment location, the first release link 24 is pulled by the surgeon to retract the first pod 20, exposing the first stent 16 to the vascular environment. The first link 24 may be attached to a handle at the proximal end of the delivery catheter 10, the surgeon pulling on the handle to retract the first pod 20. Alternately, the first link 24 may be wound around a rotary handle at the proximal end of the delivery catheter 10, the surgeon rotating the rotary handle to retract the first pod 20. In one embodiment, a combination of these two actions may independently manipulate the release links, for example a first release link 24 is activated by rotating the handle to wind the first link 24 around the handle. A second link 26 is connected to the same handle but not affected by the rotation thereof. However, an axial displacement of the handle may activate the second link 26.

Being free from the constraint of the first pod 20, the first stent 16 expands to its deployed diameter, and engages the wall of the vessel in which it is deployed. Preferably, the first stent 16 comprises a shape memory material which reacts to the heat of the vascular blood at body temperature by expanding to a deployed diameter. Additionally, delivery catheter 10 preferably also comprises a cylindrical outer lumen (not shown) which can enclose the distal structure shown in Fig. 1 during insertion, and be retracted for deployment. Accordingly, the first pod 20 is retracted into the outer lumen when first stent 16 is deployed.

Alternately or additionally, the expansion of the stent 16 following the displacement of the pod 20 may be delayed by the prior application of a restraint holding the stent 16, 18 to the central core 12. The restraint may be released by degradation of the restraining material, appropriately selected from one those known for such a purpose. Alternately or additionally, the restraint may be released by the severing the restraint. For example, the first release link 24, or a separate dedicated release link, may pass beneath the restraint and over the stent 16. Additional tension on the release link 24 following the displacement of pod 20 from over stent 16, or any tension on the separate release link dedicated for that purpose, would rupture the restraint, and free the stent 16 to expand to its deployed diameter. This delayed release arrangement will be seen as equally applicable to second stent 18, and optionally second release link 24 as discussed, *infra.*

In the embodiment where first pod 20 is withdrawn from over the first stent 16, friction at the interface between the pod 20 and the stent 16 may be reduced by the application of a coating to the interior of the first pod 20. Such coatings may comprise silicone, MDX, or a hydrophilic material, among others suitable for the purpose. Optionally, the first stent 16 can be coated for drug delivery. In that case, an anti-friction coating on an interior of the pod 20 can avoid removing stent coating during retraction of the pod 20 by friction between the pod 20 and the first stent 16.

In an alternate embodiment, the release link 24 may run under the first pod 20 along some or all of its length. Applying tension to the release link 24 would pull the link through the material of the first pod 20, rupturing it, and release the pod 20 from around the first stent 16. The release link 24 could be bound to the material of the first pod 20 to draw it into an outer lumen as in the previous embodiment. Alternately, the pod may comprise a bioabsorbable material, which disintegrates into the bloodstream after release. Alternately, the pod material may be biocompatible and/or implantable, and be pressed between the exterior of stent 16 and the interior of the surrounding tissue without any negative implications. This deployment method has the advantage that it does not have to overcome friction between the pod 20 and the first stent 16.

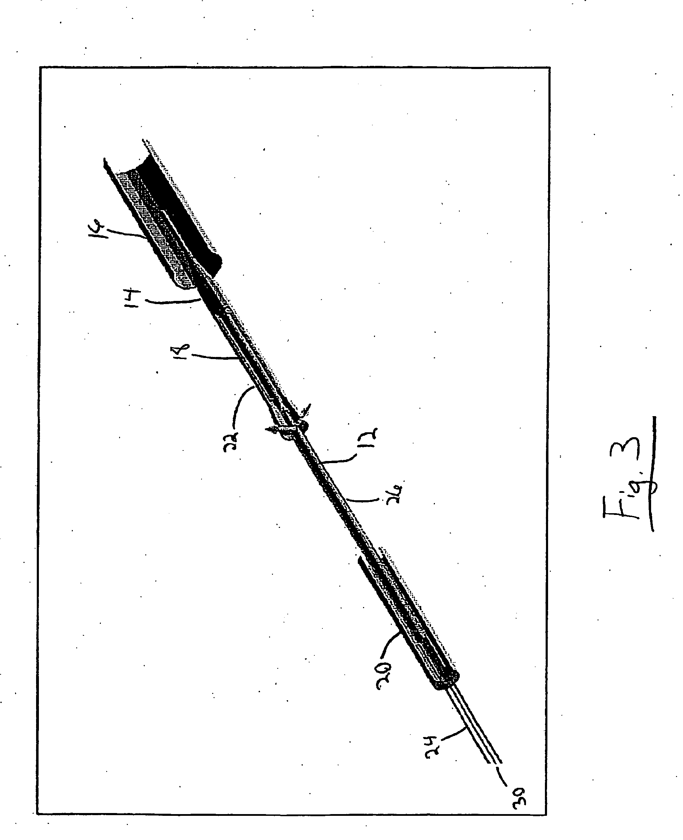

Referring now to Fig. 3, following deployment of first stent 16, the delivery catheter 10 is repositioned by retracting it through the deployed first stent 16 in the proximal direction. Therefore, there is no risk of distal or cranial migration by interference with the delivery catheter 10. The second stent 18 is located in its deployment position.

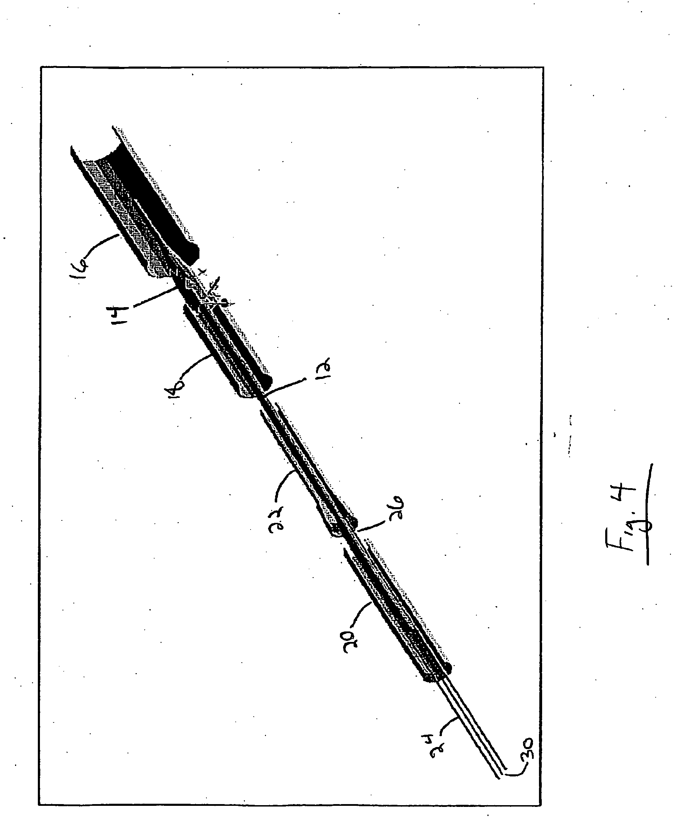

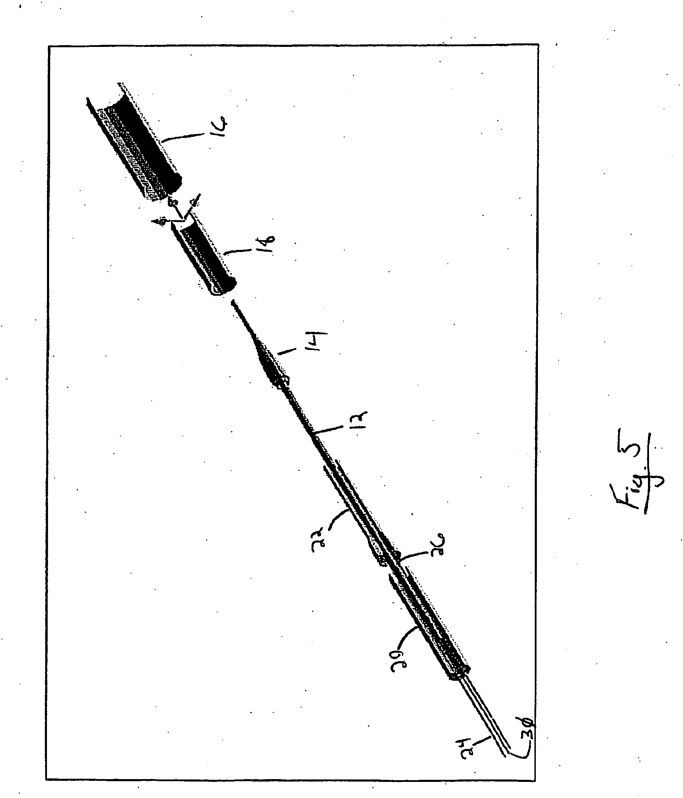

Referring now to Fig. 4, tension is applied to the second release link 26 to withdraw or alternately break through second pod 22, as described generally with respect to the first pod 20 and the release of the first stent 16. The second stent 18 is then free to expand to its deployed diameter within the vessel. It will be appreciated that the manner of releasing the second pod 22 may be by withdrawing the second pod 22 or breaking through the second pod 22, irrespective of the manner of releasing the first pod 20. Referring to Fig. 5, the delivery catheter 10 is withdrawn in the proximal direction from within stents 16, 18 and the vessel in which they were deployed.

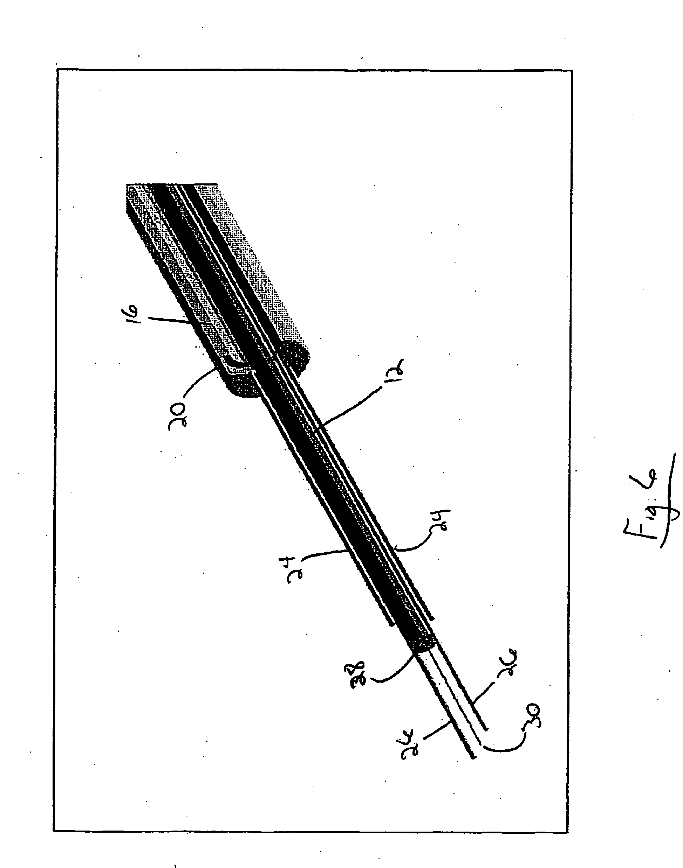

Referring now to Fig. 6, illustrates is a detail view of the delivery catheter 10 at the proximal end of first pod 20, in partial cutaway view. First stent 16, as well as first and second release links 24, 26 are visible. Also shown is a cross section of the central core 12, which includes an axial lumen 28 which can admit a guide wire 30 to assist in inserting and locating the delivery catheter 10 for deployment of stents 16, 18.

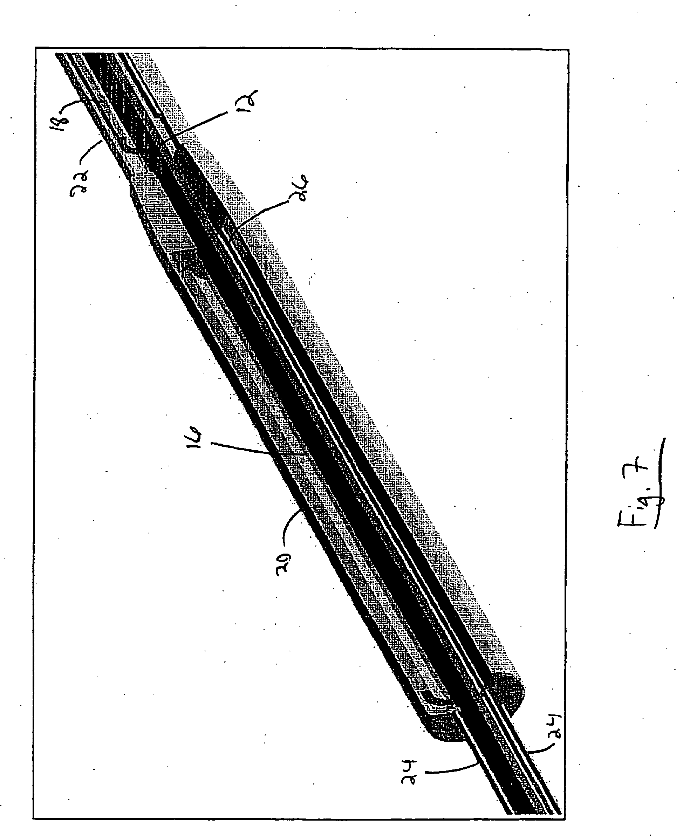

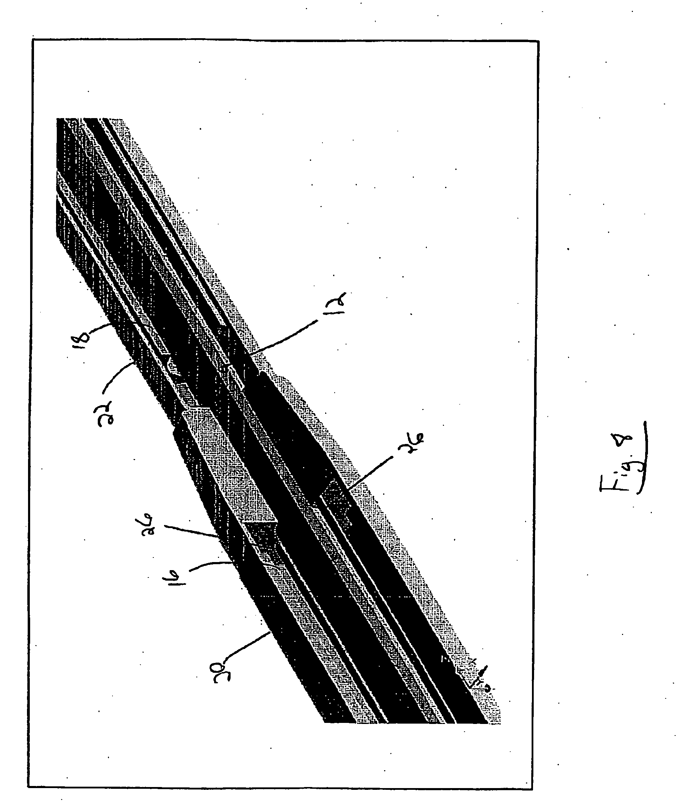

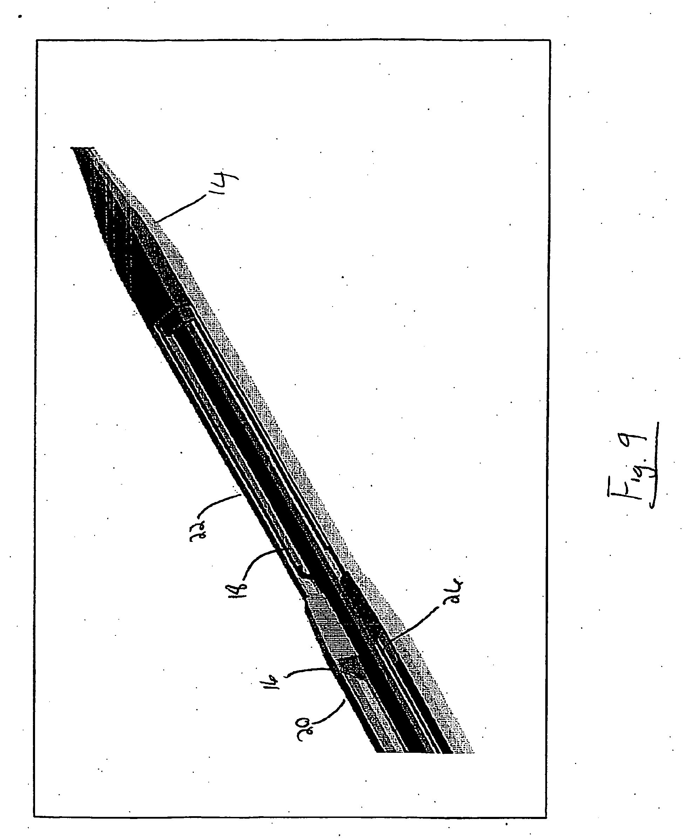

Referring now to Figs. 7 and 8, illustrated is the interface between the first pod 20 and the second pod 22. Pods 20 and 22 are preferably linked to one another so that the delivery catheter 10 may be rotated as a unit during insertion. Moreover, it is preferred that the transition between first pod 20 and second pod 22 be continuous on its outer surface to reduce resistance from the surrounding vessel upon insertion of the delivery catheter 10. Referring now to Fig. 9, illustrated is the interface between the second pod 22 and the distal tip 12 of the delivery catheter 10. The interface between the second pod 22 and the distal tip 14 of the delivery catheter 10 is also preferably continuous, again to reduce resistance from the surrounding vessel on insertion.

The delivery catheter 10 having a central lumen 28 for guide wire 30 will by recognized by those skilled in the art as an over-the-wire type configuration. Alternately, however, the distal tip 12 of the delivery catheter 10 may include an abbreviated passage to accept the guide wire 30, as part of a so-called rapid-exchange design as is known in the art. Accordingly, the delivery catheter 10 need not be threaded over the entire length of the guide wire, and the guide wire can be shorter. Moreover, using a rapid-exchange design obviates the need for a central lumen to admit the guide wire through all or most of its length. Accordingly, the overall diameter of the delivery catheter can be advantageously reduced. In certain embodiments of the present invention, whether rapid exchange design or over-the-wire; the size of the delivery catheter can be reduced to permit percutaneous insertion, eliminating the need for incision and the associated risks and drawbacks to the patient. Since the point of connection in the rapid-exchange design is distal of the pods 20, 22, the guide wire 30 would necessarily be outside the prosthesis after deployment, to be subsequently withdrawn.

In yet another alternate embodiment, the first pod 20 and first release link 24 can be dispensed with, and instead the outer lumen as described, *supra,* can maintain the first stent 16 in position before deployment. Then, the first stent 16 can be deployed by axially displacing the outer lumen with respect to the distal region of the delivery catheter 10, uncovering the first stent 16. Considered in another way, the lumen itself extending from the proximal region of the delivery catheter 10 to the stent 16, can comprise both the pod and the link. Similarly, an intermediate lumen may comprise both the pod and the link associated with the distal stent 18.

According to preferred embodiments of the present invention, a single delivery catheter delivers plural implants. This reduces the time required to perform the implantation procedure, reducing stress and trauma to the patient. The implantation is also simpler, requiring a single delivery catheter to deliver the plural implants. The implantation is also easier to perform, reducing the opportunity for errors and then demands on the skill of the surgeon.

The present invention has been described herein with reference to certain exemplary or preferred embodiments. These embodiments are offered as merely illustrative, not limiting, of the scope of the present invention. For example, the delivery catheter 10 herein disclosed may be used to deliver more than two stents. Other applications for the present invention include the use of one or more stents to open an occluded vessel, commonly a coronary artery, particularly where a vessel may be subject to multiple blockages along its length.

Alternately, the present invention is applicable for the delivery of longer and/or serial stent grafts to other parts of the body, for example as used in the treatment of Superficial Femoral Artery aneurysm (SFA). Moreover, the terms 'stent' or 'stent graft' as used throughout the foregoing disclosure should be construed in a generic fashion, and encompass a variety of implantable medical devices, particularly tubular and/or prosthetic implants. For example, the present invention is applicable for the delivery of shunts or vascular grafts not having stents.

## Claims

1. A delivery catheter (10) for the deployment of a medical implant device comprising:
a generally cylindrical central core (12);
a distal tip (14) of the delivery catheter at a distal end of the central core;
first and second generally cylindrical pods (20, 22) for covering one or more prosthetic implants (16, 18) located around the central core (12),
first and second independent release links (24, 26), each extending from a proximal region of the delivery catheter (10) to an interface with the first and second pods (20, 22), respectively, and operative to displace the first and second pod (20, 22), respectively,
**characterised in that** the first and second pods (20, 22) are connected with one another to move as a unit and to present a continuous outer surface;
the first pod (20) is located proximally of the second pod (22);
the second pod (22) is connected with the distal tip (14) to move as a unit and to present a continuous outer surface; and wherein the second independent release link (26) comprises one or more of a solid filament, a hollow filament, and a braided filament.

2. The delivery catheter according to claim 1, wherein the first or second independent release links (24, 26) comprises one or more of a metal or plastic material.

3. The delivery catheter according to claim 1, wherein the first or second independent release links (24, 26) is secured to the respective first or second pod (20, 22) at a proximal end of the first or second pod (20, 22).

4. The delivery catheter according to claim 1, wherein the first or second independent release links (24, 26) pass within a respective first or second pod (20, 22), and configured to respond to tension applied to the first or second release link (24, 26) by rupturing the exterior of a respective first or second pod (20, 22).

5. The delivery catheter according to claim 4, wherein the first or second pod (20, 22) ruptured by the respective first or second release link (24, 26) comprises one or more of a biocompatible, bioimplantable, and bioabsorbable material.

6. The delivery catheter according to claim 1, further comprising one or more user-operable handles at a proximal end of the delivery catheter, wherein a proximal end of first or second independent release links (24, 26) are connected to the one or more handles, and manipulating the one or more handles applies tension to the first or second release links (24, 26).

7. The delivery catheter according to claim 6, wherein at least one of the one or more handles are rotary handles, and manipulating the rotary handle comprises rotating it.

8. The delivery catheter according to claim 1, further comprising an axial lumen (28) through at least a portion of the central core (12).

9. The delivery catheter according to claim 1, further comprising a rapid exchange connection for removing or interchanging a portion of the delivery catheter.

10. The delivery catheter according to claim 1, further comprising an outer lumen sized to enclose the distal region of the delivery catheter, and axially displaceable with respect to the distal region of the delivery catheter.

11. The delivery catheter according to claim 1, further comprising a biocompatible lubricant material at an inner surface of the first or second pods (20, 22).

12. The delivery catheter according to claim 1, wherein one or more of the first and second release links (24, 26) is operative to rupture a restraint constraining a prosthetic implant to be delivered by the delivery catheter.

13. The delivery catheter according to claim 1, further comprising a third independent release link operative to rupture a restraint constraining a prosthetic implant to be delivered by the delivery catheter.

## Patentansprüche

1. Zuführungskatheter (10) zur Einbringung einer medizinischen Implantationsvorrichtung, welche Folgendes umfasst:
einen im Allgemeinen zylindrischen, zentralen Kern (12);
eine distale Spitze (14) des Zuführungskatheters an einem distalen Ende des zentralen Kerns;
eine erste und eine zweite im Allgemeinen zylindrische Hülse (20, 22), um ein oder mehrere prosthetische Implantate (16, 18), welche sich um den zentralen Kern (12) herum befinden, abzudecken,
eine erste und eine zweite unabhängige Freigabeverbindung (24, 26), die sich jeweils von einem proximalen Bereich des Zuführungskatheters (10) zu einer Schnittstelle mit der ersten bzw. zweiten Hülse (20, 22) erstrecken und wirksam sind, um die erste bzw. zweite Hülse (20, 22) zu verschieben,
**dadurch gekennzeichnet, dass** die erste und zweite Hülse (20, 22) miteinander verbunden sind, um sich als eine Einheit zu bewegen, und um eine durchgehende äußere Oberfläche zu bilden;
die erste Hülse (20) sich proximal zur zweiten Hülse (22) befindet;
die zweite Hülse (22) mit der distalen Spitze (14) verbunden ist, um sich als eine Einheit zu bewegen, und um eine durchgehende äußere Oberfläche zu bilden; und wobei die zweite unabhängige Freigabeverbindung (26) ein festes Filament, ein hohles Filament und/oder ein geflochtenes Filament umfasst.

2. Zuführungskatheter nach Anspruch 1, wobei die erste oder zweite unabhängige Freigabeverbindung (24, 26) ein Material oder mehrere Materialien aus Metall oder Kunststoff umfasst.

3. Zuführungskatheter nach Anspruch 1, wobei die erste oder zweite unabhängige Freigabeverbindung (24, 26) an der zugehörigen ersten oder zweiten Hülse (20, 22) an einem proximalen Ende der ersten oder zweiten Hülse (20, 22) befestigt ist.

4. Zuführungskatheter nach Anspruch 1, wobei die erste oder zweite unabhängige Freigabeverbindung (24, 26) innerhalb einer zugehörigen ersten oder zweiten Hülse (20, 22) verläuft, und so eingerichtet ist, dass sie auf Spannung reagiert, welche an der ersten oder zweiten Freigabeverbindung (24, 26) ausgeübt wird, indem sie die Außenfläche einer zugehörigen ersten oder zweiten Hülse (20, 22) aufreißt.

5. Zuführungskatheter nach Anspruch 4, wobei die erste oder zweite Hülse (20, 22), welche durch die zugehörige erste oder zweite Freigabeverbindung (24, 26) aufgerissen wird, ein oder mehrere biokompatible, bioimplantierbare und/oder bioabsorbierbare Materialien umfasst.

6. Zuführungskatheter nach Anspruch 1, welcher weiterhin einen oder mehrere durch einen Anwender zu bedienende Griffe an einem proximalen Ende des Zuführungskatheters umfasst, wobei ein proximales Ende der ersten oder zweiten unabhängigen Freigabeverbindung (24, 26) mit dem Griff verbunden ist, und eine Betätigung des einen oder der mehreren Griffe eine Spannung auf die erste oder zweite Freigabeverbindung (24, 26) ausübt.

7. Zuführungskatheter nach Anspruch 6, wobei zumindest einer der Griffe ein Drehgriff ist, und ein Betätigen des Drehgriffs ein Drehen desselben umfasst.

8. Zuführungskatheter nach Anspruch 1, welcher weiterhin ein axiales Lumen (28) durch zumindest einen Teilbereich des zentralen Kerns (12) umfasst.

9. Zuführungskatheter nach Anspruch 1, welcher weiterhin einen Anschluss zum schnellen Austausch umfasst, um einen Teil des Zuführungskatheters zu entfernen oder auszuwechseln.

10. Zuführungskatheter nach Anspruch 1, welcher weiterhin ein äußeres Lumen umfasst, welches so bemessen ist, dass es den distalen Bereich des Zuführungskatheters umfasst, und axial gegenüber dem distalen Bereich des Zuführungskatheters verschiebbar ist.

11. Zuführungskatheter nach Anspruch 1, welcher weiterhin ein biokompatibles Gleitmaterial auf einer inneren Oberfläche der ersten oder zweiten Hülse (20, 22) umfasst.

12. Zuführungskatheter nach Anspruch 1, wobei eine oder mehrere der ersten und zweiten Freigabeverbindungen (24, 26) wirksam ist, um eine Einspannung aufzureißen, welche ein prosthetisches Implantat einspannt, welches mittels des Zuführungskatheters zugeführt werden soll.

13. Zuführungskatheter nach Anspruch 1, welcher weiterhin eine dritte unabhängige Freigabeverbindung aufweist, welche wirksam ist, um eine Einspannung aufzureißen, welche ein prosthetisches Implantat einspannt, welches mittels des Zuführungskatheters zugeführt werden soll.

## Revendications

1. Cathéter de pose (10) pour le déploiement d'un dispositif d'implant médical, comprenant :
une âme centrale (12) généralement cylindrique ;
une pointe distale (14) du cathéter de pose au niveau d'une extrémité distale de l'âme centrale ;
des première et seconde nacelles (20, 22) généralement cylindriques pour recouvrir un ou plusieurs implants prothétiques (16, 18) situés autour de l'âme centrale (12),
des première et deuxième liaisons de libération indépendantes (24, 26), chacune s'étendant à partir d'une région proximale du cathéter de pose (10) vers une interface avec les première et seconde nacelles (20, 22), respectivement, et opérationnelles pour déplacer les première et seconde nacelles (20, 22) respectivement,
**caractérisé en ce que** les première et seconde nacelles (20, 22) sont raccordées l'une à l'autre pour se déplacer comme une unité afin de présenter une surface externe continue ;
la première nacelle (20) est située à proximité de la seconde nacelle (22) ;
la seconde nacelle (22) est raccordée avec la pointe distale (14) pour se déplacer comme une unité et pour présenter une surface externe continue ; et dans lequel la deuxième liaison de libération indépendante (26) comprend un ou plusieurs parmi un filament plein, un filament creux et un filament tressé.

2. Cathéter de pose selon la revendication 1, dans lequel la première ou la deuxième liaison de libération (24, 26) indépendante comprend un ou plusieurs parmi un métal ou une matière plastique.

3. Cathéter de pose selon la revendication 1, dans lequel la première ou la deuxième liaison de libération (24, 26) indépendante est fixée à la première ou seconde nacelle (20, 22) respective au niveau d'une extrémité proximale de la première ou de la seconde nacelle (20, 22).

4. Cathéter de pose selon la revendication 1, dans lequel la première ou la deuxième liaison de libération (24, 26) indépendante passe à l'intérieur d'une première ou seconde nacelle (20, 22) respective et est configurée pour répondre à une tension appliquée sur la première ou la deuxième liaison de libération (24, 26) en rompant l'extérieur d'une première ou seconde nacelle (20, 22) respective.

5. Cathéter de pose selon la revendication 4, dans lequel la première ou la seconde nacelle (20, 22) rompue par la première ou deuxième liaison de libération (24, 26) respective comprend un ou plusieurs parmi un matériau biocompatible, un matériau bioimplantable et un matériau bio-absorbable.

6. Cathéter de pose selon la revendication 1, comprenant en outre une ou plusieurs poignées pouvant être actionnées par un utilisateur au niveau d'une extrémité proximale du cathéter de pose, dans lequel les extrémités proximales des première ou deuxième liaisons de libération (24, 26) indépendantes sont raccordées à une ou à plusieurs poignées, et une manipulation des une ou plusieurs poignées applique une tension sur les première ou deuxième liaisons de libération (24, 26).

7. Cathéter de pose selon la revendication 6, dans lequel au moins l'une des une ou plusieurs poignées est une poignée rotative, et la manipulation de la poignée rotative comprend sa rotation.

8. Cathéter de pose selon la revendication 1, comprenant en outre une lumière axiale (28) à travers au moins une partie de l'âme centrale (12).

9. Cathéter de pose selon la revendication 1, comprenant en outre un raccordement à échange rapide pour retirer ou changer une partie du cathéter de pose.

10. Cathéter de pose selon la revendication 1, comprenant en outre une lumière externe dimensionnée pour entourer la région distale du cathéter de pose, et axialement déplaçable par rapport à la région distale du cathéter de pose.

11. Cathéter de pose selon la revendication 1, comprenant en outre un matériau lubrifiant biocompatible au niveau d'une surface interne de la première ou de la seconde nacelle (20, 22).

12. Cathéter de pose selon la revendication 1, dans lequel une ou plusieurs parmi les première et deuxième liaisons de libération (24, 26) sont opérationnelles pour rompre une retenue contraignant un implant prothétique destiné à être posé par le cathéter de pose.

13. Cathéter de pose selon la revendication 1, comprenant en outre une troisième liaison de libération indépendante opérationnelle pour rompre une retenue contraignant un implant prothétique destiné à être posé par le cathéter de pose.
